# EUROPEAN PATENT APPLICATION

(11) **EP 3 418 271 A1**
(43) Date of publication of application: **26.12.2018**
(21) Application number: 17753430.2
(22) Date of filing: 13.02.2017
(51) Int. Cl.: C07D 209/24, C07D 209/26, C07D 405/12, A61K 31/404, A61K 31/343, A23L 33/10, A61K 8/49, A61Q 19/00

(54) **NOVEL INDOLE DERIVATIVE AND ANTI-CANCER COMPOSITION CONTAINING SAME**

(30) Priority: 19.02.2016 KR 20160019968
(71) Applicant: Korea Research Institute of Bioscience and Biotechnology, Daejeon 34141 (KR)
(72) Inventor: KIM, Bo Yeon, Daejeon 34141 (KR); SOUNG, Nak Kyun, Daejeon 34141 (KR); GANIPISETTI, Srinivasrao, Daejeon 34141 (KR); AHN, Jong Seog, Daejeon 34141 (KR); JANG, Jae-Hyuk, Daejeon 34141 (KR); KO, Sung-Kyun, Daejeon 34141 (KR); RYOO, In Ja, Daejeon 34141 (KR); CHA, Hyunjoo, Daejeon 34141 (KR); HWANG, Joon Sung, Daejeon 34141 (KR); LEE, Kyung Ho, Daejeon 34141 (KR)
(74) Representative: Dehns
(86) International application number: PCT/KR2017/001542
(87) International publication number: WO 2017/142269

(57) **Abstract**

The present invention relates to: an indole derivative, which is a novel cell mitosis inhibitor; a stereoisomer thereof or a pharmaceutically acceptable salt thereof; a use thereof as a therapeutic agent; a composition containing the same and a treatment method using the composition; and a preparation method therefor. According to the present invention, the indole derivative, the stereoisomer thereof or the pharmaceutically acceptable salt thereof, inhibits the tubulin polymerization during mitosis so as to induce apoptosis, and has an excellent anti-cancer effect also in cancer cells having multiple drug resistance.

## Description

### Technical Field

The present invention relates to a novel indole derivative compound and an anti-cancer composition comprising the same. Specifically, the present invention relates to a novel indole derivative compound showing excellent anti-cancer effect, low toxicity, and excellent solubility, and an anti-cancer composition comprising the same.

### Background Art

Microtubules perform a number of cellular functions including cell migration, cell division, cytoskeletal retention, and intracellular transport. The main protein component of the microtubule is tubulin. The rapid growth of cancer cells depends on tubulin polymerization/depolymerization significantly, making tubulin an excellent target in developing anti-cancer agents. Intervention with microtubule assemblies by inhibiting tubulin polymerization or inhibiting microtubule degradation increases the number of cells collected in the metaphase and eventually causes apoptosis. Using tubulin-targeting drugs to inhibit the microtubule's function is an acknowledged approach in chemotherapy.

Drugs that target microtubules can be divided into two groups depending on their action mechanisms: microtubule stabilizers and destabilizers. Microtubule stabilizers include taxane, paclitaxel, docetaxel, etc., which serves the function of inhibiting depolymerization of microtubules and enhancing polymerization of microtubules. Most microtubule stabilizers bind to either the taxane binding site or the overlapping site of β-tubulin. The second group, microtubule destabilizers, includes colchicine, vinca alkaloid, etc., which inhibit polymerization of microtubules, and mostly binds to the colchicine binding site or vinca binding site. In addition, the two groups of drugs targeting microtubules work at lower concentrations than drugs that affect microtubule polymers.

However, most tubulin inhibitors have the problem of drug resistance, which is a major obstacle in enhancing long-term responses or survival rates of cancer patients. In addition, a neurotoxicity problem as well as the resistance problem is one of the major side effects of tubulin inhibitors derived from complex natural products, which affect the quality of life of cancer patients. Furthermore, low oral-bioavailability poses limitation to comfortable oral administration. Therefore, there is an urgent need, recently, to develop new tubulin inhibitors that show low side effects but excellent oral bioavailability, and do not lead to drug resistance easily.

### Disclosure of Invention

### Technical Problem

An object of the present invention is to provide a novel tubulin inhibitor showing an anti-cancer effect and an anti-cancer composition comprising the same.

Another object of the present invention is to provide a novel tubulin inhibitor showing an excellent anti-cancer effect, a low toxicity and an excellent solubility, and an anti-cancer composition comprising the same.

### Solution to Problem

In order to achieve the object of the present invention, the present invention provides a novel indole derivative compound having tubulin-inhibitory activity, a stereoisomer thereof, or a pharmaceutically acceptable salt thereof. The present invention also provides an anti-cancer composition comprising an indole derivative compound of the present invention, a stereoisomer thereof, or a pharmaceutically acceptable salt thereof as an active ingredient. Hereinafter, the present invention will be described in detail.

### Novel indole derivative compound

The present invention provides an indole derivative compound represented by Formula I below, a stereoisomer thereof, or a pharmaceutically acceptable salt thereof:

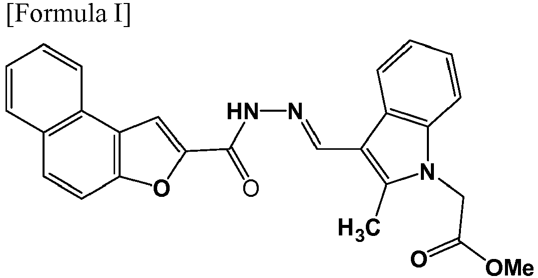

In the present invention, the pharmaceutically acceptable salt refers to a salt generally used in a pharmaceutical industry. Examples of the pharmaceutically acceptable salt include inorganic ion salts formed with calcium, potassium, sodium, magnesium, etc.; inorganic acid salts formed with hydrochloric acid, nitric acid, phosphoric acid, bromic acid, iodic acid, perchloric acid, sulfuric acid, etc.; organic acid salts formed with acetic acid, trifluoroacetic acid, citric acid, maleic acid, succinic acid, oxalic acid, benzoic acid, tartaric acid, fumaric acid, mandelic acid, propionic acid, lactic acid, glycolic acid, gluconic acid, galacturonic acid, glutamic acid, glutaric acid, glucuronic acid, aspartic acid, ascorbic acid, carbonic acid, vanillic acid, hydroiodic acid, etc.; sulfuric acid salts formed with methanesulfonic acid, ethanesulfonic acid, benzenesulfonic acid, p-toluenesulfonic acid, naphthalene sulfonic acid, etc.; amino acid salts formed with glycine, arginine, lysine, etc.; and amine salts formed with trimethylamine, triethylamine, ammonia, pyridine, picoline, etc. However, the types of the salts of the present invention are not limited to the salts listed above.

The compounds represented by Formula I may contain one or more asymmetrical carbon atoms, and thus may exist in the form of racemates, racemic mixtures, single enantiomers, diastereomeric mixtures, and individual diastereomers. The compounds of formula I can be separated into such isomers by methods known in the art, for example, column chromatography or HPLC. Alternatively, stereoisomers of the compounds of formula I may be synthesized by stereospecific synthesis using optically pure starting materials and/or reagents of known configuration.

The indole derivative represented by Formula I, a stereoisomer thereof, or a pharmaceutically acceptable salt thereof according to the present invention induces apoptosis of cells by inhibiting tubulin polymerization during cell mitosis, and has an excellent anti-cancer effect also in cancer cells having multiple drug resistance, and thus it can be effectively used as an anti-cancer agent.

Further, the indole derivative represented by Formula I, a stereoisomer thereof, or a pharmaceutically acceptable salt thereof according to the present invention shows excellent stability *in vivo*, and excellent solubility, and thus exhibits excellent bioavailability.

### Anti-cancer composition comprising novel indole derivative compound, use thereof, and therapeutic method using the same

The present invention also provides an anti-cancer composition comprising an indole derivative compound represented by Formula I, a stereoisomer thereof, or a pharmaceutically acceptable salt thereof as an active ingredient.

The indole derivative compound represented by Formula I, a stereoisomer thereof, or a pharmaceutically acceptable salt thereof showed an anti-proliferative activity in various cancer cell lines, even in cancer cell lines showing multiple drug resistance. Accordingly, a composition comprising an indole derivative compound represented by Formula I, a stereoisomer thereof, or a pharmaceutically acceptable salt thereof as an active ingredient can be used as a pharmaceutical composition for preventing and treating cancer.

In the present invention, the cancer may be selected from the group consisting of rectal cancer, breast cancer, lung cancer, gastric cancer, liver cancer, leukemia, glioma, skin cancer and cervical cancer. However, the present invention is not limited thereto.

The pharmaceutical composition of the present invention may further comprise at least one active ingredient having anti-cancer activity.

The pharmaceutical composition of the present invention can further comprise a pharmaceutically acceptable additive, which is exemplified by starch, gelatinized starch, microcrystalline cellulose, lactose, povidone, colloidal silicon dioxide, calcium hydrogen phosphate, lactose, mannitol, taffy, Arabia rubber, pregelatinized starch, corn starch, cellulose powder, hydroxypropyl cellulose, Opadry, sodium carboxy methyl starch, carunauba wax, synthetic aluminum silicate, stearic acid, magnesium stearate, aluminum stearate, calcium stearate, white sugar, dextrose, sorbitol, talc, etc. The pharmaceutically acceptable additive of the present invention is preferably added to the composition in an amount of 0.1 to 90 parts by weight, but is not limited thereto.

The pharmaceutical composition according to the present invention may be formulated as an oral or parenteral preparation for actual clinical administration, in which case the generally-used filling agents, extenders, binders, wetting agents, disintegrants, diluents such as surfactants, or excipients may be used. The solid formulation for oral administration may include tablets, pills, powders, granules, capsules, etc., and may be prepared by mixing with at least one excipient such as starch, calcium carbonate, sucrose, lactose or gelatin. Further, a lubricant such as magnesium stearate, and talc may be used in addition to the simple excipient. Examples of liquid formulations for oral administration include a suspension, liquid for internal use, an emulsion, a syrup, etc. In addition to typically used diluents such as water, and liquid paraffin, various excipients such as a wetting agent, a sweetening agent, a flavoring agent, a preservative, and the like may be included. Examples of preparations for parenteral administration may include a sterilized solution, a nonaqueous solvent, a suspension, an emulsion, a lyphophilized preparation, and suppository. As the nonaqueous solvent and suspending solvent, propylene glycol, polyethylene glycol, vegetable oil such as olive oil, injectable ester such as ethyl oleate may be used. As a substrate for the suppository, witepsol, macrogol, tween 61, cacao oil, laurinum, glycerogelatin, etc. may be used.

The composition of the present invention may be orally administered or parenterally administered according to the desired method. For parenteral administration, topical skin administration, intraperitoneal injection, intrarectal injection, subcutaneous injection, intravenous injection, intramuscular injection or intrathoracic injection methods are preferably used. The dosage can vary depending on weight, age, sex, health status, and diet of a patient, administration time, administration methods, excretion rates, and severity of a disease.

The composition according to the present invention is administered in a pharmaceutically effective amount. In the present invention, "a pharmaceutically effective amount" refers to an amount sufficient to treat a disease with a reasonable benefit/risk ratio available to apply a medicinal treatment. The level of effective dose may be determined depending on factors including types of diseases of patients, severity, activity of a drug, sensitivity to a drug, administration time, administration routes, excretion rates, period of treatment, and a simultaneously used drug, and other factors well known in the medicinal field. The composition of the present invention may be administered as a separate therapeutic agent or administered in combination with other therapeutic agents. Also, the composition of the present invention may be sequentially or simultaneously added with a conventional therapeutic agent, and the composition may be used for single or multiple administrations. It is important to administer at a minimal dose which may lead to a maximum effect without side effects in consideration of all of the factors described above, and the doses may be easily determined by a person skilled in the art.

Specifically, an effective amount of the compound according to the present invention may vary depending on age, sex, and weight of a patient. Generally, 0.1 mg to 100 mg, and preferably, 0.5 mg to 10 mg per 1 kg of body weight may be administered daily or every other day, or administered 1 to 3 times per day. However, the dose may be increased or decreased depending on administration routes, severity of obesity, sex, weight, age, and so forth, and thus the above dose does not limit the scope of the present invention in any way.

The present invention also provides a use of an indole derivative compound represented by Formula I, a stereoisomer thereof, or a pharmaceutically acceptable salt thereof, for use in preventing or treating cancer.

The present invention also provides a use of an indole derivative compound represented by Formula I, a stereoisomer thereof, or a pharmaceutically acceptable salt thereof, for use in the manufacture of a drug for preventing or treating cancer.

The present invention also provides a method for preventing or treating cancer, comprising administering to a subject in need of preventing or treating cancer a therapeutically effective amount of an indole derivative compound of Formula I, a stereoisomer thereof, or a pharmaceutically acceptable salt thereof. In the present invention, the "subject" includes mammals, particularly humans.

In addition, the indole derivative represented by Formula I, a stereoisomer thereof, or a pharmaceutically acceptable salt thereof according to the present invention shows an excellent anti-proliferative activity, and thus a composition comprising the same as an active ingredient can be effectively used as a food composition for preventing or improving cancer.

The food composition of the present invention may further contain a conventional additive used in other food composition, health functional food, or beverage.

For instance, the food composition of the present invention can contain sweetening agents such as sucrose, granulated fructose, glucose, D-sorbitol, mannitol, isomaltooligosaccharide, stevioside, aspartame, acesulfame potassium, sucralose, and the like, acidifiers such as anhydrous citric acid, DL-malic acid, succinic acid and its salt, and the like, preservatives such as benzoic acid and its derivative, and the like, various nutritional supplements, vitamins, minerals (electrolyte), flavoring agents such as synthetic and natural flavoring agents, coloring agents, flavor enhancers (cheese, cholate and the like), pectic acid and its salt, alginic acid and its salt, organic acid, protective colloid thickeners, pH modifiers, stabilizers, preservatives, glycerin, alcohol, carbonating agents used for carbonated beverage, and the like. Also, the food composition of the present invention can contain pulp for preparation of natural fruit juice and vegetable beverage. The ratio of these additives can be decided within the range of about 20 parts by weight or below based on 100 parts by weight of the food composition of the present invention.

When the food composition of the present invention is a beverage, it can further comprise flavoring agents or natural carbohydrate commonly contained in beverages. The natural carbohydrate can be a monosaccharide such as glucose and fructose, a disaccharide such as maltose and sucrose, a polysaccharide such as dextrin and cyclodextrin, or sugar alcohol such as xylitol, sorbitol and erythritol. In addition, the flavoring agent can be a natural flavoring agent such as thaumatin, stevia extract (rebaudioside A, glycyrrhizin and the like), or a synthetic flavoring agent such as saccharin, aspartame and the like. When the food composition is prepared as a beverage, natural carbohydrate can be contained generally in an amount of about 1 to 20 g, and preferably about 5 to 12 g based on 100 mL of the composition.

The food composition of the present invention can be prepared in a form of powders, granules, a tablet, a capsule or a beverage, and be used as food, a beverage, a gum, a tea, a vitamin complex and health supplement foods.

In addition, an indole derivative represented by Formula I, a stereoisomer thereof, or a pharmaceutically acceptable salt thereof according to the present invention showed an excellent anti-cancer activity in a human-derived skin cancer cell line (A432) implantation mouse model, the composition containing the same as an active ingredient can be effectively used as a cosmetic composition for preventing or improving skin cancer.

The composition of the present invention can be formulated as any preparations that are generally formulated in the art, which are exemplified by solution, suspension, emulsion, paste, gel, cream, lotion, powders, soap, surfactant-containing cleansing, oil, powdered foundation, emulsified foundation, wax foundation, spray, etc., but is not limited thereto. More specifically, the composition of the present invention can be formulated as soft lotion, nutrition lotion, nutrition cream, massage cream, essence, eye cream, cleansing cream, cleansing foam, cleansing water, pack, spray or powder.

When the cosmetic composition according to the present invention is formulated as paste, cream or gel, animal oil, vegetable oil, wax, paraffin, starch, tragacanth, a cellulose derivative, polyethylene glycol, silicone, bentonite, silica, talc, zinc oxide, etc. may be used as a carrier ingredient.

When the cosmetic composition according to the present invention is formulated as powders or a spray, lactose, talc, silica, aluminum hydroxide, calcium silicate or polyamide powders may be used. In particular, a spray may further contain a propellant such as chlorofluorohydrocarbon, propane/butane or dimethyl ether.

When the cosmetic composition according to the present invention is formulated as a solution or an emulsion, a solvent, a solubilizer or an emulsifier may be used as a carrier ingredient. For example, water, ethanol, isopropanol, ethyl carbonate, ethyl acetate, benzyl alcohol, benzyl benzoate, propylene glycol, 1,3-butylene glycol, an aliphatic glycerol ester, polyethylene glycol or a fatty acid ester of sorbitan may be used.

When the cosmetic composition according to the present invention is formulated as a suspension, a liquid diluent such as water, ethanol or propylene glycol, a suspending agent such as ethoxylated isostearyl alcohol, polyoxyethylene sorbitol ester and polyoxyethylene sorbitan ester, microcrystalline cellulose, aluminum metahydroxide, bentonite, agar, tragacanth, etc. may be used as a carrier ingredient.

When the cosmetic composition according to the present invention is formulated as a surfactant-containing cleanser, an aliphatic alcohol sulfate, aliphatic alcohol ether sulfate, sulfosuccinic acid monoester, isethionate, an imidazolinium derivative, methyl taurate, sarcosinate, a fatty acid amide ether sulfate, an alkylamidobetaine, an aliphatic alcohol, a fatty acid glyceride, a fatty acid diethanolamide, a vegetable oil, a lanolin derivative, an ethoxylated glycerol fatty acid ester, etc. may be used as a carrier ingredient.

### Advantageous Effects of Invention

An indole derivative, a stereoisomer thereof, or a pharmaceutically acceptable salt thereof according to the present invention induces apoptosis of cells by depolymerization of microtubules during cell mitosis, and has an excellent anti-cancer effect also in cancer cells having multiple drug resistance. In addition, the indole derivative, the stereoisomer thereof or the pharmaceutically acceptable salt thereof of the present invention exhibits excellent stability *in vivo* and an excellent solubility.

### Brief Description of Drawings

Fig. 1 shows the effect of the compound of Example 1 (WCI-1031) on the spindle fibers and chromosomes of the mitotic cells depending on the concentration of the compound examined by immunofluorescence staining.
Fig. 2 is a graph showing changes in weights after treatment with the compound in a human-derived skin cancer cell line (A431) implantation nude mouse model.
Fig. 3 is a graph showing changes in tumor sizes after treatment with the compound in a human-derived skin cancer cell line (A431) implantation nude mouse model.
Fig. 4 provides photographs showing the tumor sizes of the mice on the last day (day 23) after treatment with the compound in a human-derived skin cancer cell line (A431) implantation nude mouse model.
Fig. 5 is a graph showing the tumor weights after treatment with the compound in a human-derived skin cancer cell line (A431) implantation nude mouse implantation model.
Fig. 6 provides photographs showing the tumors isolated from the mice on the last day (day 23) after treatment with the compound in a human-derived skin cancer cell line (A431) implantation nude mouse model.
Fig. 7 is a graph showing the changes in plasma concentration over time after intravenous administration of the compound of Example 1 (WCI-1031).
Fig. 8 is a solubility curve of the compound of Example 1 (WCI-1031) at pH 6.5.
Fig. 9 is a solubility curve of the compound of Example 1 (WCI-1031) at pH 7.4.

### Best Mode for Carrying out the Invention

### Mode for the Invention

Hereinafter, the present invention is explained in detail by Examples. The following Examples are intended to further illustrate the present invention without limiting its scope.

### <Example 1> Preparation of methyl (E)-2-(2-methyl-3-((2-(naphtho[2,1-b]furan-2-carbonyl) hydrazono)methyl)-1H-indole-1-yl)acetate (WCI-1031)

Naphtho[2,1-b]furan-2-carbohydrazide (100 mg, 0.44 mmol) and methyl 2-(3-formyl-2-methyl-1H-indol-1-yl)acetate (102 mg, 0.44 mmol) and a solution of a catalytic amount of acetic acid (0.1 ml) in ethanol (5 ml) were stirred at 90°C for 2 hours. After confirming that the starting substances were exhausted by TLC, the reaction mixture was cooled to room temperature and added to ice water. The separated solid mass was filtered, washed with water and then dried. The residue thus produced was purified by silica gel flash column chromatography using a mixture of hexane and ethyl acetate at a mixing ratio of 1: 1, to obtain the desired compound, methyl (E)-2-(2-methyl-3-((2-(naphtho[2,1-b]furan-2-carbonyl) hydrazono)methyl)-1H-indole-1- yl)acetate (174 mg, 90%) as a pale yellow solid.
ESIMS found: m/z 440.36[M+H]⁺, 879.16[2M+H]⁺.

### <Experimental Example 1> Examination of anti-proliferative activity of indole derivative of the present invention

### <1-1> Examination of anti-proliferative activity in HeLa cell line

The following experiments were conducted to examine the anti-proliferative activity of the indole derivatives of the present invention.

HeLa cells (ATCC, USA), which are a human cervical cancer cell line, were dispensed into a 96-well plate at 2 x 10³ cells/well, and the cells were treated with DMSO or the indole derivatives of the present invention, and then allowed to grow for 4 days. And then, MTT(3-(4,5-Dimethylthiazol-2-yl)-2,5-diphenyltetrazolium bromide) reagent was added to the wells at 10 µl/well. After 2 hours, the absorbance was measured at "OD 450" and statistical values were obtained using the plizm6 program. The data represent mean values of the results of two repeated assays (Table 1).

**[Table 1]**

| Treatment substance | | IC₅₀ (µM) |
|---|---|---|
| DMSO | | - |
| Example 1 | WCI-1031 | 0.653 |

As shown in Table 1, the indole derivatives of the present invention were found to have excellent anti-proliferative activity in the HeLa cell line (cervical cancer cell line).

### <1-2> Examination of anti-proliferative activity in other cancer cell lines

The following experiment was conducted to determine whether the indole derivative of the present invention shows anti-proliferative activity in other cancer cells as well as in the HeLa cell line (cervical cancer cell line).

Specifically, various cancer cell lines were cultured on a microtiter plate (1-3 x 10³ cells/well), and the cells were treated with the indole derivative of the present invention and cultured for 4 days. Cytotoxicity was examined by the MTT assay by the same method as in Experimental Example 1-1, and the IC₅₀ was obtained by the log-dose response curve. The data represent mean values of the results of three repeated assays (Table 2).

**[Table 2]**

| Cell line | Origin of Tumor | Example 1 |
|---|---|---|
| | | WCI-1031 |
| HeLa | Uterine cercix | 0.297 |
| Hep3B | Liver | 0.503 |
| HepG2 | Liver | 2.402 |
| A172 | Brain | 1.117 |
| U373MG | Brain | 0.262 |
| A431 | Skin | 0.186 |
| MCF7 | Breast | 1.321 |
| PC3 | Prostate | 0.549 |
| NCIH-125 | Lung | 0.333 |
| NCIH-460 | Lung | 0.671 |
| NCIH-1299 | Lung | 0.376 |
| A 549 | Lung | 0.495 |
| SNU 484 | Stomach | 0.493 |
| Wehi 3 | Bone marrow | 0.290 |
| K562 | Bone marrow | 0.549 |

As shown in Table 2, it was found that the indole derivative of the present invention has an excellent anti-proliferative activity in various cancer cell lines as well.

### <1-3> Examination of anti-proliferative activity in cancer cells showing multiple drug resistance

The following experiment was conducted to examine whether the indole derivatives of the present invention are effective in cancer cells showing multiple drug resistance.

Specifically, K562 and MCF7 (Bio Evaluation Center, Korea Research Institute of Bioscience and Biotechnology, Korea), and K562/ADR and MCF7/ADR (Bio Evaluation Center, Korea Research Institute of Bioscience and Biotechnology, Korea), which are multiple drug-resistant cell lines of the above mentioned cell lines, respectively, were cultured on a microtiter plate at 1-3 x 10³ cells/well. Then, the cells were treated with WCI-1031 (compound of Example 1), an indole derivative of the present invention, taxol, doxorubicin, vinblastine, or colchicine, and cultured for 4 days. The cytotoxicity was examined by the MTT assay by the same method as in Experimental Example 1-1, and the IC₅₀ was obtained by the log-dose response curve (unit: nM). The data represent mean values of the results of three repeated assays (Tables 3 and 4).

The resistance factor of a cell line exhibiting multiple drug resistance is the ratio of the IC₅₀ of the multiple drug-resistant cell line to the IC₅₀ of a non-resistant parent cell line.

**[Table 3]**

| | WCI-1031 | Taxol | Doxorubicin | Vinblastine | colchicine |
|---|---|---|---|---|---|
| K562 | 532 | 1.347 | 2.848 | 17.13 | 15.86 |
| K562/ADR | 317.8 | 699.9 | 2187 | 267.8 | 363.4 |
| Resistance factor | 0.60 | 519.6 | 767.91 | 15.63 | 22.91 |

**[Table 4]**

| | WCI-1031 | Taxol | Doxorubicin | Vinblastine | colchicine |
|---|---|---|---|---|---|
| MCF7 | 1321 | 2.345 | 15.38 | 5.275 | 2.71 |
| MCF7/ADR | 481.1 | 1028 | 2608 | 95.87 | 92.54 |
| Resistance factor | 0.36 | 438.38 | 169.57 | 18.17 | 34.15 |

As shown in the Tables 3 and 4, the multiple drug-resistant cell lines exhibited strong resistance to the conventional anti-cancer agents, with resistance factors ranging from several tens to several hundreds. However, the resistance factors against the compound of Example 1, an indole derivative of the present invention, ranged from 0.36 to 0.60, indicating that the indole derivative of the present invention has a stronger cytotoxic effect on the cancer cells which show multiple drug resistance, as compared to the conventional anti-cancer agents,.

### <Experimental Example 2> Examination of effect of indole derivative of the present invention on cell cycle progression

Several cancer cell lines were cultured on a 12 well plate (3 x 10⁴ cells/well), and treated with DMSO or WCI-1031, an indole derivative of the present invention, for 17 hours, and then added with a propidium iodine dye to stain the cellular DNA, and subjected to measurement using FACS. The concentrations at which cells gathered in the G2/M phase and numbers of the cells represented as percentages were shown (Table 5).

**[Table 5]**

| Cell line | effect µM (% of G2/M) |
|---|---|
| HeLa | 0.5 (82.36) |
| Hep3B | 0.5 (68.13) |
| A172 | 1.0 (70.10) |
| A431 | 0.5 (66.32) |
| MCF7 | 0.5 (59.08) |
| MCF7 ADR | 0.5 (52.37) |
| PC3 | 0.5 (65.35) |
| NCIH-125 | 1.0 (48.10) |
| EL4 | 0.5 (50.84) |
| K562 | 0.5 (76.72) |

With regard to WCI-1031, an indole derivative of the present invention, cells gathered in the G2/M phase at the concentrations of 0.5 to 1.0 µM, and the numbers of the cells were 50% or higher, as shown in Table 5. Therefore, it was found that the indole derivative of the present invention suppresses the cell cycle in G2 and M phases.

### <Experimental Example 3> Examination of effect of indole derivative of the present invention on tubulin polymerization

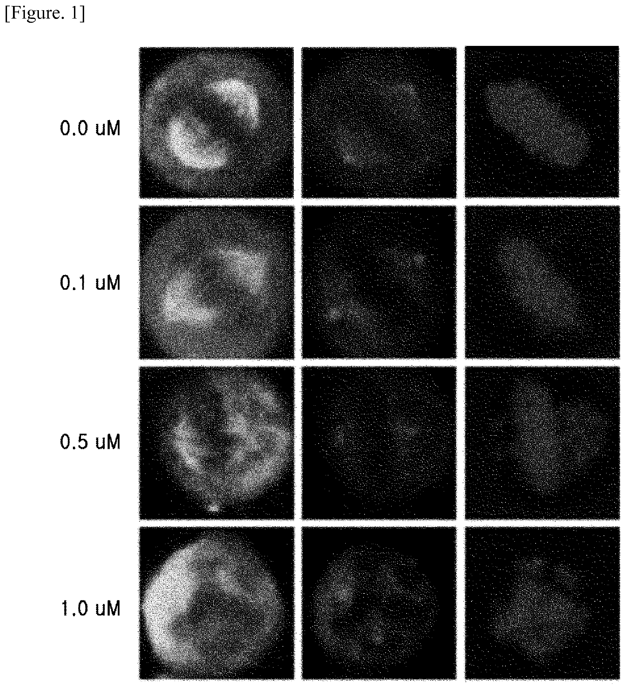

In order to examine the effect of the indole derivative of the present invention on intracellular microtubules, HeLa cells were treated with DMSO or WCI-1031 (0.1 µM, 0.5 µM, and 1.0 µM) for 16 hours. The cells were fixed, and stained with an anti-tubulin antibody and Alexa Fluor 488, and stained with an anti-centrosome antibody and Texas Red, and then the nuclei of the cells were immunostained using Hoechst 33342 to examine the α-tubulin, centrosome and DNA (Fig. 1).

As shown in Fig. 1, when the cells were treated with the indole derivative of the present invention, as the concentration of the derivative became higher, the shape of the tubulin became loose and shorter, as compared to the DMSO control. And the pattern of the DNA which deviated from the central array was increasingly found.

Therefore, it was found that the indole derivative of the present invention is a formulation which depolymerizes microtubules.

### <Experimental Example 4> Examination of anti-cancer effect in a human-derived skin cancer cell (A431) implantation model

### <4-1> Culture of cancer cells and implantation of cancer cells

The human cancer cell line A431 which was kept frozen in liquid nitrogen was thawed and cell culture was conducted. Cells were cultured in a CO₂ incubator (Forma, USA) at 37°C and 5% CO₂ for an appropriate period of time.

On the last day of culture, all cancer cells were collected and counted and the cell concentration was adjusted to 1 x 10⁷ cells/ml using serum free media. The culture solution thus prepared was injected subcutaneously in the axillary region between the scapula and chest wall in an amount of 0.3 ml per BALB/C female nude mouse (5 weeks old, Nara Biotech) (3 x 10⁶ cells/mouse).

### <4-2> Method of preparation and administration of samples

WCI-1031 (Example 1), an indole derivative compound of the present invention, was used as a test substance, and 5-FU and colchicine were used as positive control substances.

The compounds were used after dissolving in DMAC(dimethyl acetamide) 20% + Tween80 5% + 20% HPbCD(2-hydroxypropyl-beta-cyclodextrin) 75% to appropriate concentrations immediately before administration. 5-FU and colchicine used as the positive control substances were prepared for use in the concentrations of 2 and 0.007 mg/ml using normal saline and PBS, respectively.

The prepared substances were repeatedly administered intraperitoneally and orally at 0.2 ml per 20 g of the mouse (10 ml/kg) according to the dosage schedule below.
-carrier, WCI-1031 (10, 20 mg/kg), colchicine (0.07 mg/kg): days 0-23
-5-FU: days 0-2, 5-9, 12-16, 19-23

### <4-3> General symptom and weight change verificaiton

To examine the toxicity levels of the repeated intraperitoneal administration of WCI-1031 to A431 cancer cell implantation nude mice, general symptoms and weight changes of the animals were observed during the administration period.

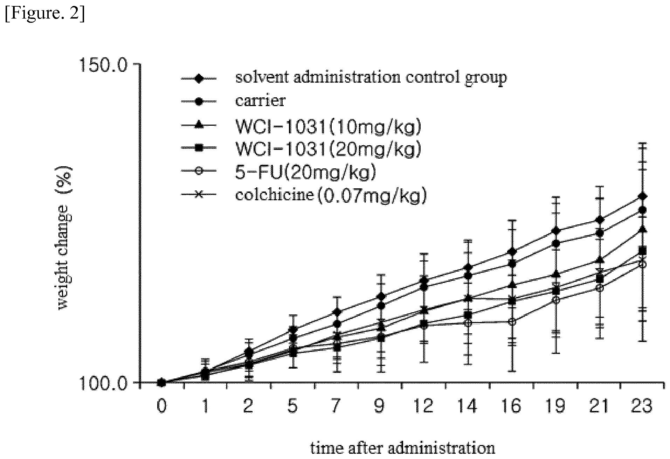

As a result, no statistically significant weight loss was observed in all drug-treated groups as compared to the solvent control group, and no significant general symptoms were observed during the test period (Fig. 2).

### <4-4> Examination of tumor size changes

After the cancer cell implantation, the tumor size of each animal was measured in three directions using a vernier caliper from the point when the average tumor size reached 57.0 mm³ until day 23, for a total of 11 times, which was expressed by the equation of length x width x height/2.

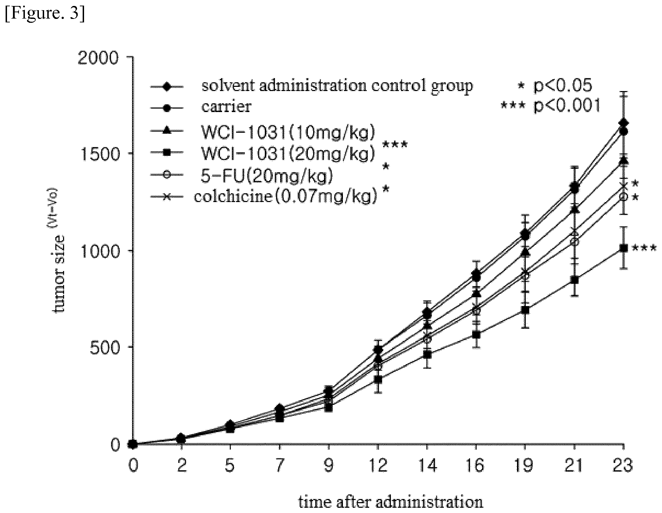

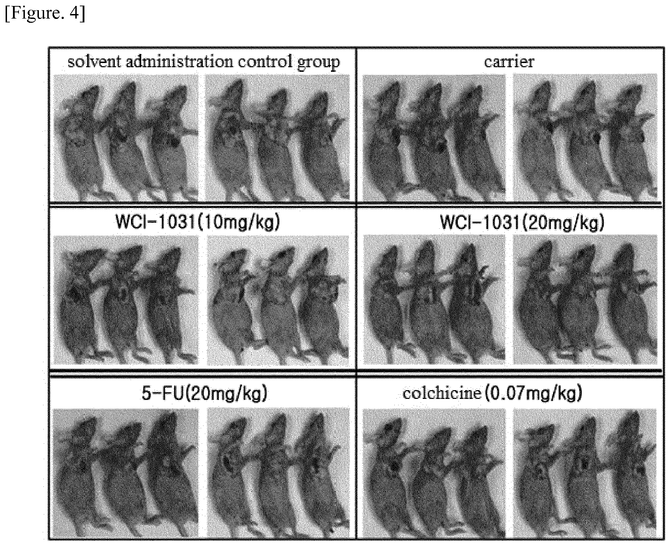

On the last day (day 23), tumor growth inhibitions of 9.3% and 37.2% (p <0.001) were observed in the groups administered with WCI-1031 at 10 and 20 mg/kg, respectively, as compared to the solvent administration control group. The tumor growth inhibitions in the 5-FU and colchicine administration groups, the positive control groups, were 20.7% (p <0.01) and 17.6% (p <0.05), respectively (Figs. 3 and 4).

### <4-5> Examination of tumor weight changes

On day 23 after the start of the drug administration, blood was taken from the mice through ophthalmic veins 2 hours after the last administration, and the mice were sacrificed using CO₂ gas. Then the mice were photographed and the tumors was isolated and weighed in a chemical balance. After taking photographs, each tumor was divided in half, and the resultant tumors were fixed to liquid nitrogen and formalin, respectively.

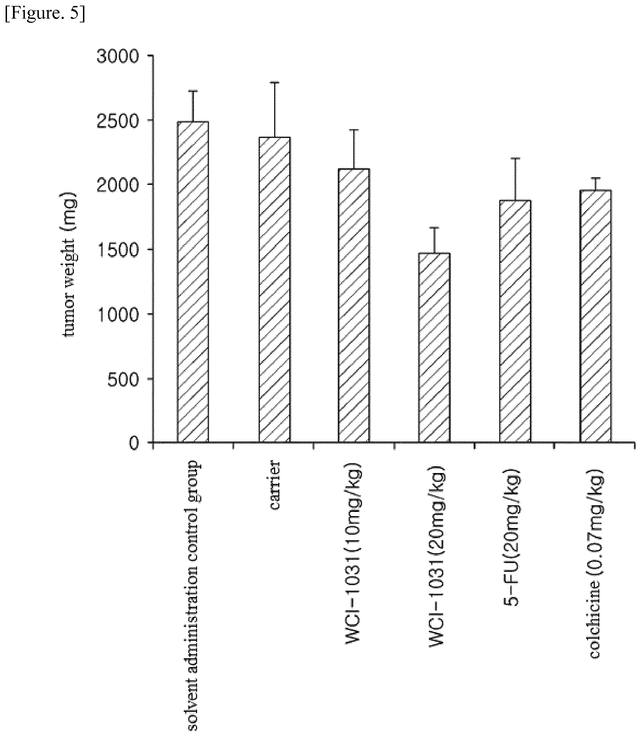

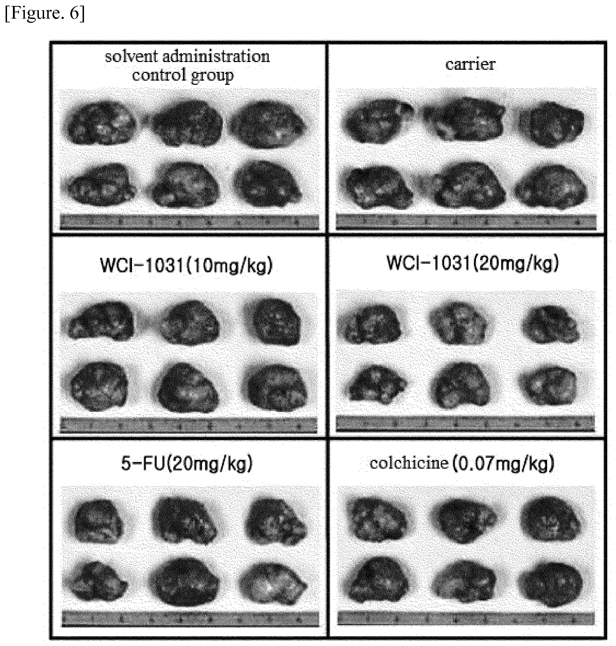

On day 16 after the start of administration of the drug, A431 tumors were excised and weighed. The tumor weight reductions of 10.7% and 38.0% (p <0.001) were observed in the groups administered with WCI-1031 at 10 and 20 mg/kg, respectively. The tumor weight reductions in the 5-FU and colchicine administration groups, the positive control groups, were 20.8% (p <0.05) and 17.6% (p <0.05), respectively (Figs. 5 and 6).

### <Experimental Example 5> Examination of stability of indole derivative of the present invention in blood plasma

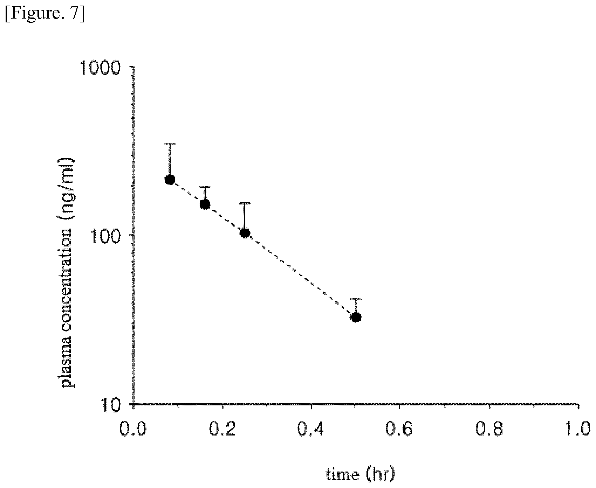

In order to examine the stability of the indole derivative of the present invention *in vivo*, the compound of Example 1 (WCI-1031) was injected intravenously and the plasma concentration was observed over time (Fig. 7).

As shown in Fig. 7, the stability of WCI-1031 in the blood plasma was found to be excellent.

### <Experimental Example 6> Examination of solubility of indole derivative of the present invention

In order to examine the solubility of the indole derivative of the present invention, the solubility of the compound of Example 1 (WCI-1031) was examined at pH 6.5 or pH 7.4.

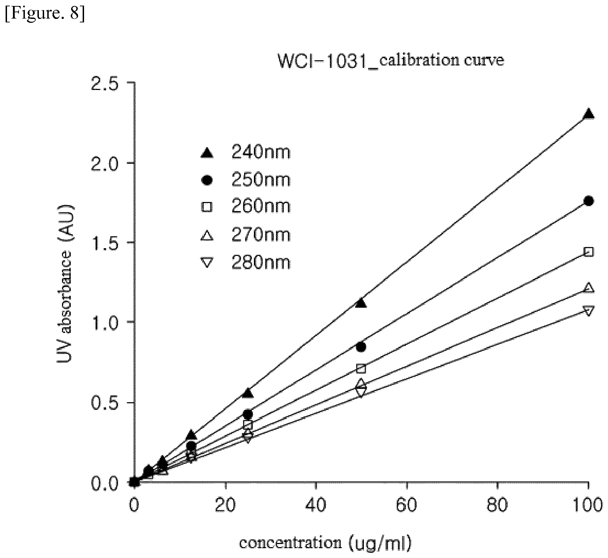

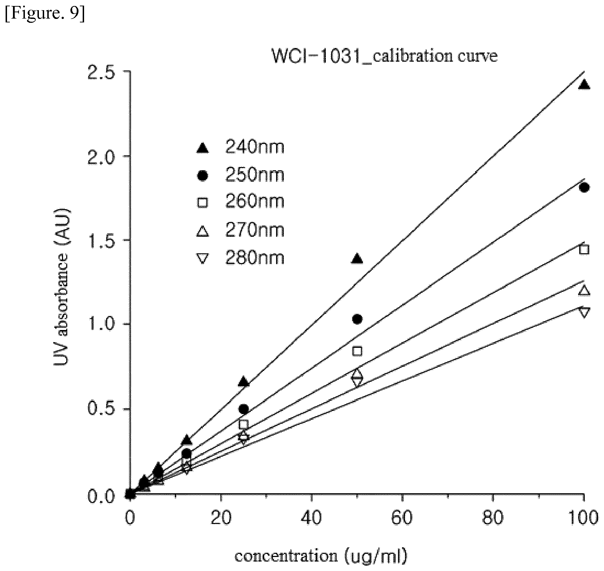

Specifically, a solid sample (∼1 mg) of the test substance was placed on Whatman Syringeless filter (PVDF membrane, 0.45 µm pore size) and mixed with 0.5 ml of a phosphate buffer (pH 6.5 or 7.4). The mixture was sonicated for 15 minutes and vortexed for 1 hour at room temperature. The equilibrated mixture was filtered and the filtrate was analyzed with a multi-wavelength UV plate reader (Figs. 8 and 9).

The data of the solubility and the like of WCI-1031 are shown in Table 6 below.

**[Table 6]**

| pH | Solubility (µg/ml) | Ka (min⁻¹) | SIWV (ml) | SITT (ml) | MAD (mg) |
|---|---|---|---|---|---|
| 6.5 | 0.9 | 0.03 | 250 | 270 | 1.8 |
| 7.4 | 0.4 | 0.03 | 250 | 270 | 0.8 |

| | | | | | |
|---|---|---|---|---|---|
| (Ka: intestinal absorption rate constant SIWV: small intestinal water volume (∼250 ml) SITT: small intestinal transit time (∼270 min) MAD : maximum absorbable dose in human) | | | | | |

## Claims

1. An indole derivative compound represented by Formula I below, a stereoisomer thereof, or a pharmaceutically acceptable salt thereof:

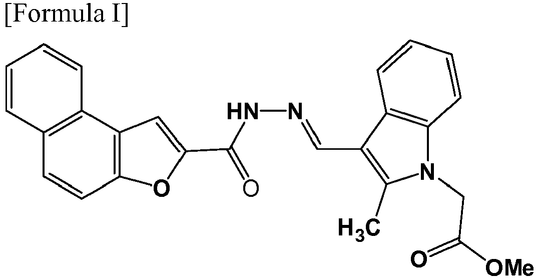

2. A pharmaceutical composition for preventing or treating cancer, comprising an indole derivative compound represented by Formula I below, a stereoisomer thereof, or a pharmaceutically acceptable salt thereof as an active ingredient:

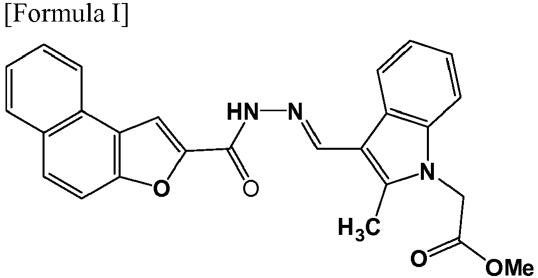

3. The pharmaceutical composition according to claim 2, wherein the cancer is selected from the group consisting of rectal cancer, breast cancer, lung cancer, gastric cancer, liver cancer, leukemia, glioma, skin cancer and cervical cancer.

4. A food composition for preventing or improving cancer, comprising an indole derivative compound represented by Formula I below, a stereoisomer thereof, or a pharmaceutically acceptable salt thereof as an active ingredient:

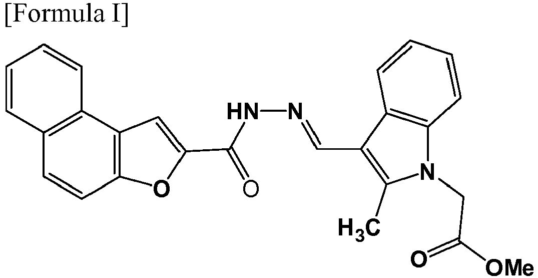

5. A cosmetic composition for preventing or improving skin cancer, comprising an indole derivative compound represented by Formula I below, a stereoisomer thereof, or a pharmaceutically acceptable salt thereof:
